# EUROPEAN PATENT SPECIFICATION

(11) **EP 1 360 945 B1**
(45) Date of publication and mention of the grant of the patent: **29.11.2006**
(21) Application number: 03101217.2
(22) Date of filing: 02.05.2003
(51) Int. Cl.: A61F 2/16, A61F 9/00, A61F 9/007

(54) **Insertion System for Intraocular Lens**
System zum Einsetzen einer Intraokularlinse
Système d'injection de lentille intraoculaire

(30) Priority: 08.05.2002 JP 2002133180
(43) Date of publication of application: 12.11.2003
(73) Proprietor: Canon-Staar Co., Inc., Tokyo (JP)
(72) Inventor: Kikuchi, Toshikazu, TOKYO (JP); Kobayashi, Kenichi, Tokyo (JP)
(74) Representative: Stanley, David William

(56) References cited:
- FR-A- 2 814 360
- US-A1- 2001 007 942
- US-A1- 2001 014 808

## Description

The present invention relates to systems for inserting a deformable intraocular lens into the eye. Examples of such a deformable intraocular lens include a deformable intraocular lens that is inserted into the eye in place of the natural lens when the latter is physically extracted because of cataracts, and a vision correction lens that is inserted into the eye for the sole purpose of vision correction.

In general, during cataract surgery, an intraocular lens is inserted into the eye, from which the natural lens has been removed (lens-removed eye), such that the intraocular lens is located in the original position previously occupied by the natural lens and restores vision. Various studies on the material and shape of such an intraocular lens have been carried out since Ridley performed the first implantation of an artificial lens in 1949.

In recent years, in addition to studies on intraocular lenses which are used for vision restoration after cataract surgery, intense studies on intraocular lenses for refractivity correction have been ongoing. Such an intraocular lens for refractivity correction is inserted into the eye which still has a natural lens (lens-carrying eye), for correction of nearsightedness or farsightedness.

In relation to cataract surgery, a technique for crushing the lens tissue by means of ultrasonic emulsification and suctioning the crushed tissue away has been widely used. This technique enables performance of lens removal surgery to excise an opaque lens through a small incision. Along with progress in the operational technique itself, intraocular lenses themselves have recently been improved. Such an improved intraocular lens is disclosed in, for example, Japanese Patent Application Laid-Open (*kokai*) No. 58-146346. In the intraocular lens, the optical portion is made of a deformable elastic material. The intraocular lens is inserted, in a folded state, into the eye through a small incision and restored to its original shape within the eye, allowing it to exert its proper lens function.

Accompanying these technical developments, the material of the optical portion of such an intraocular lens has been changed gradually from hard polymethyl methacrylate (PMMA) to silicone or soft acrylic resin, which enables the intraocular lens to be inserted into the eye in a folded state.

Moreover, in recent years, studies have been conducted on copolymers such as hydroxyethyl methacrylate and methyl methacrylate, as well as on hydrophilic materials such as 2-hydroxyethyl methacrylate (HEMA).

Further, intraocular lenses of different shapes have been studied and put into practical use, including an intraocular lens having a circular optical portion and loop-shaped support portions formed of different materials, an intraocular lens whose loop-shaped support portions and optical portion are formed of the same material, and an intraocular lens having plate-shaped support portions.

Furthermore, the following patent publications disclose insertion devices for inserting the above-described deformable intraocular lens into the eye in a compressed or folded state.
(1) Japanese Patent Application Laid-Open (*kokai*) No. 5-103803 discloses a device designed such that a holding member which holds a folded lens is attached to a main body, and the lens is inserted into the eye through an insertion tube provided at the tip end of the holding member.
(2) Japanese Patent Application Laid-Open (*kokai*) No. 7-23991 discloses a disposable insertion device for one-time use in which a portion for holding a folded lens is integrated with a main body of the device and the entirety of the device is formed of resin.
(3) Japanese *Kohyo* (PCT) Patent Publication No. 9-506285 discloses an intraocular-lens insertion device having a broadened range of applications. In the intraocular-lens insertion device, a lens is held in a stress-free state in an intermediate preparation region of a main body. After attachment of a cannulae (insertion tube) to the main body, the intraocular lens is inserted into the eye through the cannulae.

The conventional intraocular-lens insertion devices described in (1) and (2) above have the following drawbacks. When either of these devices is used, an intraocular lens removed from a package is placed on a placement portion of the device, is deformed, and is then inserted into the eye. Therefore, during actual operation, work for placing the intraocular lens onto the device is needed, resulting in increased time and labour involved in implantation of the intraocular lens.

Further, such an intraocular lens and insertion device must be made germ-free through a sterilization procedure, because they are inserted into the eye through an incision. However, if an operator accidentally drops the lens and/or the insertion device onto an unclean surface, such as a floor or table, during the placement operation, the germ-free state is lost, and the lens and/or the insertion device becomes unusable.

Further, when the operator forcedly inserts into the eye an intraocular lens which has been placed on the device improperly, the lens may be broken, or may forcibly fly out from the insertion tube, potentially resulting in damage to the internal tissue of the eye.

The intraocular-lens insertion device described in (3) above has the following drawbacks. Although the intermediate region of the device can be used as a lens package, work for attaching a cannulae (insertion tube) to the main body must be performed during actual use, because the cannulae (insertion tube) is a member which is formed separately from the main body. Although the patent publication discloses a technique for storing in advance an intraocular lens at the intermediate region located on the centre axis of a push rod, it is difficult to form the intermediate region from a material suitable for storing the lens. In addition, when the lens has loop-shaped support portions, the push rod interferes with and pushes one of the support potions before coming into contact with the optical portion of the lens, thereby to deform the optical portion.

FR 2 814 360 discloses an intraocular-lens insertion device in which a lens has a support portion that engages a slot at the end of a pusher rod in a standby position. Pressure is applied to the edge of the optical portion of the lens to deform the lens ready for insertion, and the support portions of the lens are also deformed at this time.

Preferred embodiments of the present invention aim to provide an insertion system for a deformable intraocular lens, which can store an intraocular lens in a state in which no stress acts on the optical portion, thereby to eliminate the necessity of a conventionally-used lens case for the intraocular lens.

Another aim is to provide an insertion system for a deformable intraocular lens, which system eliminates or simplifies an operation of placing a lens on an insertion device thereby to save the time involved in the placement operation, while solving drawbacks involved in conventional insertion systems, such as breakage of a lens or improper insertion of a lens, which would otherwise be caused by an improper operation by an operator, and breakage of a lens support portion, which would otherwise be caused by the push rod.

Still another aim is to provide an insertion system for a deformable intraocular lens, which system enables supply of an intraocular lens and an insertion device in a sterilized state.

According to one aspect of the present invention, there is provided an insertion system for an intraocular lens having a deformable optical portion and at least one loop-shaped support portion for supporting the optical portion within an eye, the system comprising:
holding means for holding the intraocular lens at a standby position in a state in which no stress acts on the optical portion of the lens;
deforming means for deforming the lens to a reduced size;
an insertion tube through which the deformed lens is inserted into the eye; and
a pusher mechanism having a push rod for pushing the lens through the insertion tube along an axis thereof, thereby to insert the lens into the eye:
wherein:
   the holding means is arranged to hold the lens in the standby position such that the optical portion of the lens is spaced from the axis of the insertion tube and the lens is spaced from the push rod, with a tip end of the push rod located between the optical portion and the support portion of the lens as viewed perpendicularly to a plane along which the optical portion extends;
   a lens moving mechanism is arranged to move the lens transversely of the plane along which the optical portion extends, from the standby position to an insertion position at which the pusher mechanism can push and insert the lens into the eye; and
   the tip end of the push rod remains out of engagement with the lens and located between the optical portion and the support portion of the lens, when the lens is moved to the insertion position by the lens moving mechanism.

In preferred embodiments of the invention having such a configuration, a placement operation for an intraocular lens can be completed by merely moving the lens from the standby position to the insertion position by the moving mechanism. This eliminates a conventionally practiced operation of removing an intraocular lens from a lens case and placing it on the insertion system.

In addition, such an insertion system may prevent deformation or breakage of the lens support portion, which would otherwise occur due to interference with the tip end of the push rod, and prevent erroneous operation involved in the placement operation thereby to improve safety.

Further, since such an insertion system is provided with a mechanism for holding an intraocular lens at the standby position in a desired state, deformation of the lens during storage can be prevented. Moreover, when packaging and sterilization for the insertion device are performed in a state in which the lens is held in the insertion device, a completely sterilized intraocular-lens insertion system can be provided.

Preferably, the lens moving mechanism comprises interengaging protrusions and depressions that define end positions of the lens moving mechanism corresponding to the standby and insertion positions of the lens.

Preferably, the holding means comprises a base member on which the lens moving mechanism is mounted and which engages elastically with an attachment portion of a main body of the insertion system.

For a better understanding of the invention, and to show how embodiments of the same may be carried into effect, reference will now be made, by way of example, to the accompanying diagrammatic drawings, in which:
Figures 1A and 1B are views showing one example of an embodiment of an intraocular-lens insertion system according to the present invention, wherein Figure 1A is a front view of an insertion device showing a state in which a lens holding member has been attached to the insertion device and the intraocular lens is located at a first or standby position, and Figure 1B is a front view of the insertion device showing a state in which the lens is located at a second or insertion position;
Figures 2A and 2B are enlarged views showing a portion of the insertion device, wherein Figure 2A shows a state in which the intraocular lens is located at the first or standby position, and Figure 2B shows a state in which the lens is located at the second or insertion position;
Figure 2C is a plane view showing the relationship between the lens holding member and a push rod; and
Figure 3A and 3B are cross-sections of a main portion of the insertion device shown in Figures 1A and 1B, wherein Figure 3A is an enlarged cross-section taken along line 3A-3A in Figure 1A, and Figure 3B is an enlarged cross-section taken along line 3B-3B in Figure 1B.

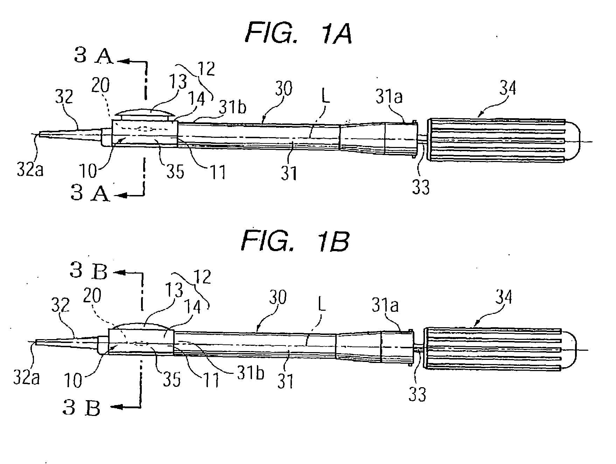

Referring to Figures 1A and 1B, an intraocular lens 20 horizontally stored in a lens holding member 10 serving as holding means for the intraocular lens 20 can be moved between a first or standby position at which the vertical position of the centre of the intraocular lens 20 does not coincide with the centre axis of a push rod 33 of an insertion device 30 and a second or insertion position at which the vertical position of the centre of the intraocular lens 20 coincides with the centre axis of the push rod 33 of the insertion device 30, so that the intraocular lens 20 can be pushed out by the push rod 33. Further, a push member 13 is provided as a lens moving mechanism for moving the intraocular lens 20 from the first or standby position to the second or insertion position.

Figure 1A is a front view of the insertion device 30 to which the lens holding member 10 has been attached and in which the intraocular lens 20 is located at the first or standby position, and Figure 1B is a front view of the insertion device 30 in which the intraocular lens 20 is located at the second or insertion position.

The system according to the present embodiment is mainly composed of the lens holding member 10, which serves as lens holding means for storing the intraocular lens 20, and the insertion device 30 for inserting the intraocular lens 20 into the eye of a patient.

The insertion device 30 includes a tubular main body 31, the above-mentioned push rod 33, a pusher mechanism 34, and an attachment portion 35. The tubular main body 31 of the insertion device 30 is formed of transparent or semi-transparent plastic or any other suitable material such that the diameter at the base end 31a is larger than that at the tip end 31b. The push rod 33 is disposed to be located on the centre axis of the tubular main body 31. The pusher mechanism 34 is disposed at the rear end 31a of the tubular main body 31 of the insertion device 30 and is coupled to the rear end of the push rod 33 so as to advance and retract the push rod 33. The attachment portion 35 is formed at the tip end 31b of the tubular main body 31 and adapted to receive the lens holding member 10 serving as holding means. A tapered insertion tube 32 is formed at the tip end of the attachment portion 35 such that the through hole of the insertion tube 32 is aligned with the centre axis of the tubular main body 31. The intraocular lens 20 is pushed out from the tip end 32a of the insertion tube 32 after being deformed to a reduced size.

In the first or standby position shown in Figure 1A, the vertical position of the centre of the lens does not coincide with the centre axis of the push rod 33 represented by an alternate long and short dash line L. The intraocular lens 20 is stored within the lens holding member 10 at the first or standby position shown in Figure 1A.

When a push member 13 of a top member 12 of the lens holding member 10 is pushed downward in Figure 1A, the intraocular lens 20 is moved downward to the second or insertion position shown in Figure 1B, at which the vertical position of the centre of the lens substantially coincides with the centre axis of the push rod 33.

In this second or insertion position, the intraocular lens 20 can be pushed out from the tip end 32a of the insertion tube 32 into the eye through advance movement of the push rod 33 effected by the pusher mechanism 34 provided at the rear end 31a of the tubular main body 31.

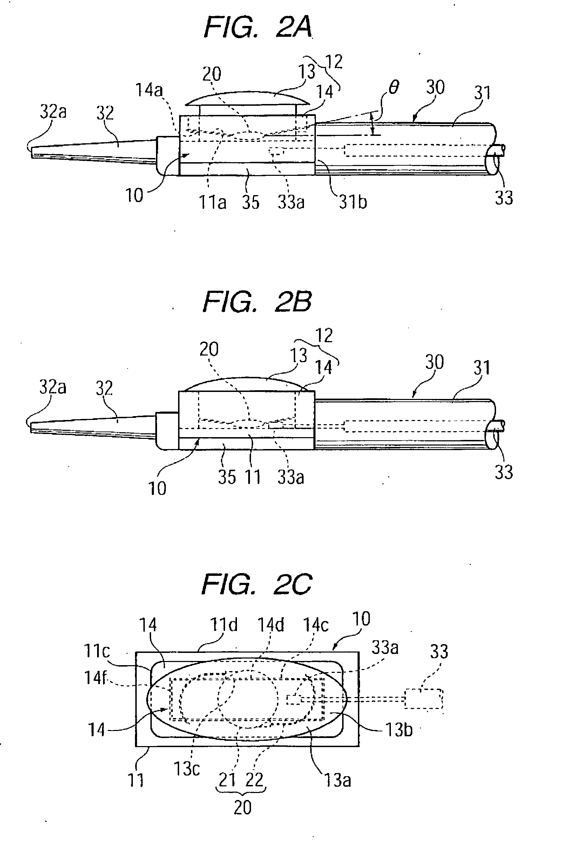

Figures 2A and 2B are views showing an assembled state in which the lens holding member 10 has been attached to the insertion device 30, wherein Figure 2A is an enlarged front view of the insertion device 30 showing a state in which the intraocular lens is located at the first or standby position, and Figure 2B is an enlarged front view of the insertion device 30 showing a state in which the lens is located at the second or insertion position. Figure 2C is an enlarged plan view of the lens holding member 10.

The lens holding member 10 consists of the above-mentioned top member 12 and a base member 11 having a structure suitable for supporting the intraocular lens 20 having loop-shaped support portions 22 made of a material different from that of the optical portion 21. Specifically, the base member 11 has engagement portions 11b which have inclined surfaces 11a of angle θ extending in opposite longitudinal directions and maintaining the angle θ between the optical portion 21 and the support portions 22 of the intraocular lens 20. A nipping member 14 of the top member 12 has on its bottom surface 14b inclined surfaces 14a to be mated with the inclined surfaces 11a of the base member 11. After placement of the lens 20 on the base member 11, the top member 12 is placed on the base member 11, so that the support portions 22 of the lens 20 are nipped between the base member 11 and the nipping member 14 of the top member 12.

A tip end 33a of the push rod 33 is located in an area between the optical portion 21 and the right-hand, loop-shaped support portion 22 as viewed from above in Figures 2A and 2B; i.e. as viewed perpendicular to a plane along which the optical portion 21 of the lens 20 extends.

Figure 2B shows a state in which the push member 13 of the top member 12 has been pushed downward, whereby the lens 20 is located at the second or insertion position. In this state, the tip end 33a of the push rod 33 is located between the optical portion 21 and the support portion 22 of the lens 20. Therefore, the tip end 33a of the push rod 33 can push the optical portion 21 of the lens 20 without interfering with the support portion 22.

This state is best seen in Figure 2C, which is a plan view of the lens holding member 10.

As is understood from Figure 2C, the tip end 33a of the push rod 33 is located in the area between the optical portion 21 and the support portion 22 from the beginning. Therefore, when the lens 20 is located at the second or insertion position as a result of the above-described operation of pressing the push member 13, the tip end 33a of the push rod 33 is located on the side toward the optical portion 21 with respect to the right-hand lens support portion 22, whereby interference between the tip end 33a with the support portion 22 can be avoided, and thus, deformation or breakage of the support portion 22 can be prevented.

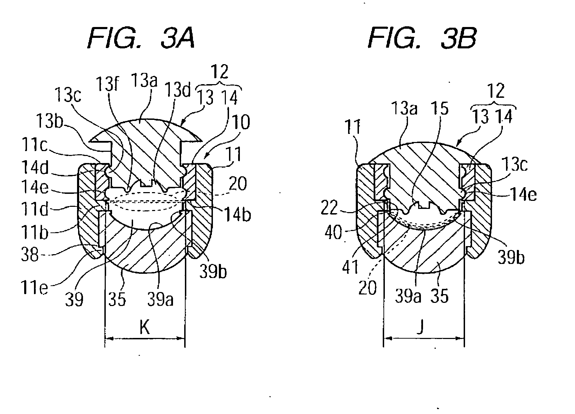

As shown in Figures 3A and 3B, the base member 11 of the lens holding member 10 has an opening 11c in the top surface thereof and projections 11e in the vicinity of the lower ends of opposite side walls 11d. The projections 11e elastically engage with engagement steps 38 formed in the vicinity of the lower ends of the lateral side surfaces of the attachment portion 35. The longitudinal opposite ends of the base member 11 are opened so that the base member 11 has a squarish C-like cross-section. Further, the paired engagement portions 11b are formed on the inner surfaces of the side walls 11d to be located at the approximate centre in the vertical direction. The engagement portions 11b extend in the longitudinal direction and are adapted to receive the peripheral portions of the optical portion 21 and the support portions 22 of the intraocular lens 20. As shown in Figure 2A, the inclined surfaces 11a each having an inclination angle θ are formed on the engagement portions 11b in order to maintain the angle θ between the optical portion 21 and the support portions 22 of the intraocular lens 20.

The top member 12 to be inserted into the top surface opening 11c of the base member 11 has the hollow nipping member 14 having a rectangular frame-like shape, and the above-mentioned push member 13 disposed in the nipping member 14 to be movable in the vertical direction. The bottom surface 14b of the nipping member 14 has the inclined surfaces 14a corresponding to the inclined surfaces 11a of the engagement portions 11b of the base member 11. Upper and lower depressions 14d and 14e are formed at a predetermined interval on each of the inner surfaces 14c of the opposite lateral walls such that the upper depressions 14d are opposed to each other and the lower depressions 14e are opposed to each other.

The above-mentioned push member 13 is inserted into the opening 14f of the nipping member 14 and is pressed downward in order to move the intraocular lens 20 from the standby position to the insertion position. The push member 13 has a head portion 13a of a large diameter and a prism-shaped leg portion 13b. Protrusions 13c are formed on the peripheral surface thereof and in the vicinity of the lower end thereof so as to be engaged selectively with the upper depressions 14d or the lower depressions 14e of the nipping member 14. Specifically, at the standby position, the protrusions 13c of the push member 13 engage the depressions 14d, and when the push member 13 is pressed, the protrusions 13c move downward and come into engagement with the depressions 14e. A concave surface 13d is formed on the bottom surface of the leg portion 13b, and a ridge 13f for supporting the peripheral portion of the intraocular lens 20 is formed on the concave surface 13d.

When the intraocular lens 20 is to be moved from the first or standby position shown in Figure 3A to the second or insertion position shown in Figure 3B, the head portion 13a of the push member 13 of the top member 12 is pressed down such that the intraocular lens 20 whose peripheral portion is partially nipped by the base member 11 and the top member 12 of the lens holding member 10 is moved to a lens movement portion 39 of the attachment portion 35. The lens movement portion 39 has a shape of a concavely-curved groove. Thus, the peripheral portion of the intraocular lens 20 comes into engagement with the reverse surfaces of the opening projection edges 39b provided at the opening of the curved concave portion 39a. As a result of this movement, the vertical position of the centre of the lens 20 coincides with the centre axis of the push rod 33 substantially. When the push rod 33 is advanced, the intraocular lens 20 is moved within the space 15 of the lens movement portion 39 in a direction perpendicular to the page of Figure 3B, passed through the insertion tube 32 provided integrally with the attachment portion 35, and is then pushed into the eye.

Since upon pressing of the push member 13 the protrusions 13c come into engagement with the depressions 14e, the intraocular lens 20 having been moved to the lens movement portion 39 is prevented from reassuming its original shape, and reliable positioning is effected.

The lens holding member 10 is preferably formed of transparent or semi-transparent material, which allows an operator to check whether the lens 20 has been moved to the lens movement portion 39.

Further, it becomes possible to check whether the space 15 for allowing movement of the intraocular lens 20 is formed between the lower surface of the top member 12 and the lens movement portion 39 of the attachment portion 35. In other words, the push member 13 of the top member 12 provides two functions; i.e. the function for moving the lens 20 downward and the function for forming the lens movement space 15 in cooperation with the attachment portion 35.

As described above, the lens holding member 10 of the embodiment - which consists of the base member 11 and the top member 12 including the nipping member 14 and the push member 13 - functions as a portion of the mechanism of the insertion device 30 upon attachment thereto.

In the above described embodiment, the tubular main body 31 of the insertion device 30 and the lens holding member 10 are assembled in order to complete the insertion device 30. However, the base member 11 may be formed integrally with the attachment portion 35 of the tubular main body 31. Further, the top member 12 may be formed integrally with the base member 11 such that the top member 12 is connected to one end portion of the upper surface of the base member 11 via a hinge.

Further, in the present embodiment, a portion of deforming means for deforming the intraocular lens 20 to a reduced size is formed integrally with the lens holding member 10.

That is, when the lens is moved to the lens movement portion 39 of the attachment portion 35, the lens is deformed to a reduced size. This size reduction is achieved by three design features; i.e. the lens movement portion 39 being formed into a form of a curved groove, the lens 20 being moved whilst being pressed toward the lens movement portion 39 by the top member 12, and the dimension J of the lens movement portion 39 being smaller than the dimension K of the lens 20.

Since such an intraocular-lens insertion device must be used in a germ-free environment, during actual use of the insertion device, an operator must use the device while wearing gloves, which hinders fine operation. Therefore, the above-described attachment method is preferable, because an operator can perform the operation of moving the intraocular lens 20 from the first or standby position to the second or insertion position by pressing the push member 13 of the lens holding member 10 from above and inserting the lens 20 from the insertion device 30 into the eye, while holding the insertion device 30, which is larger and easier to hold than the lens holding member 10.

In the above-described embodiment, the lens holding member 10 and the insertion tube 32 form deforming means for deforming the intraocular lens 20. However, embodiments of the present invention are not limited thereto, and the configuration of the device may be modified to assume various configurations; e.g. a configuration such that only the lens holding member 10 is used to deform the intraocular lens 20 to a small size suitable for insertion into the eye, and the thus-deformed lens 20 is passed through the insertion tube 32 and inserted into the eye; and a configuration such that deforming means is not provided on the lens holding member 10, but is provided on the insertion tube 32.

In this specification, the term "centre of the intraocular lens 20" refers to the centre in the thickness direction located on the optical axis of the optical portion 21.

Obviously, numerous modifications and variations of the present invention are possible in light of the above teachings. It is therefore to be understood that within the scope of the appended claims, the present invention may be practiced otherwise than as specifically described herein.

In this specification, the verb "comprise" has its normal dictionary meaning, to denote non-exclusive inclusion. That is, use of the word "comprise" (or any of its derivatives) to include one feature or more, does not exclude the possibility of also including further features.

## Claims

1. An insertion system for an intraocular lens (20) having a deformable optical portion (21) and at least one loop-shaped support portion (22) for supporting the optical portion (21) within an eye, the system comprising:
holding means (10) for holding the intraocular lens (20) at a standby position in a state in which no stress acts on the optical portion (21) of the lens (20);
deforming means (10,32) for deforming the lens (20) to a reduced size;
an insertion tube (32) through which the deformed lens (20) is inserted into the eye;
a pusher mechanism (34) having a push rod (33) for pushing the lens (20) through the insertion tube (32) along an axis thereof, thereby to insert the lens (20) into the eye:
**characterised in that**:
the holding means (10) is arranged to hold the lens (20) in the standby position such that the optical portion (21) of the lens (20) is spaced from the axis of the insertion tube (32) and the lens (20) is spaced from the push rod (33), with a tip end (33a) of the push rod (33) located between the optical portion (21) and the support portion (22) of the lens (20) as viewed perpendicularly to a plane along which the optical portion (21) extends;
a lens moving mechanism (13) is arranged to move the lens (20) transversely of the plane along which the optical portion (21) extends, from the standby position to an insertion position at which the pusher mechanism (34) can push and insert the lens (20) into the eye; and
the tip end (33a) of the push rod (33) remains out of engagement with the lens (20) and located between the optical portion (21) and the support portion (22) of the lens (20), when the lens (20) is moved to the insertion position by the lens moving mechanism (13).

2. An insertion system according to claim 1, wherein the lens moving mechanism (13) comprises interengaging protrusions (13c) and depressions (14d, 14e) that define end positions of the lens moving mechanism (13) corresponding to the standby and insertion positions of the lens (20).

3. An insertion system according to claim 1 or 2, wherein the holding means (10) comprises a base member (11) on which the lens moving mechanism (13) is mounted and which engages elastically (11e, 38) with an attachment portion (35) of a main body (31) of the insertion system.

## Patentansprüche

1. Einführungssystem für eine Intraokular-Linse (20) mit einem verformbaren optischen Abschnitt (21) und mindestens einem schlaufenförmigen Trägerabschnitt (22) zum Unterstützen des optischen Abschnitts (21) innerhalb eines Auges, wobei das System umfaßt:
ein Haltemittel (10) zum Halten der Intraokular-Linse (20) an einer Bereitschaftsposition in einem Zustand, in dem keine Spannung auf den optischen Abschnitt (21) der Linse (20) wirkt;
ein Verformungsmittel (10,32) zum Verformen der Linse (20) auf eine verringerte Größe;
einen Einführungstubus (32), durch welchen die verformte Linse (20) in das Auge eingeführt wird;
einen Schiebermechanismus (34) mit einer Schiebestange (33) zum Schieben der Linse (20) durch den Einführungstubus (32) entlang einer seiner Achsen, wodurch die Linse (20) in das Auge eingeführt wird,
**dadurch gekennzeichnet, daß**
das Haltemittel (10) angeordnet ist, um die Linse (20) in der Bereitschaftsposition derart zu halten, daß der optische Abschnitt (21) der Linse (20) von der Achse des Einführungstubus (32) beabstandet ist und daß die Linse (20) von der Schiebestange (33) beabstandet ist, wobei ein spitzes Ende (33a) der Schiebestange (33) zwischen dem optischen Abschnitt (21) und dem Trägerabschnitt (22) der Linse (20) angeordnet ist, wenn senkrecht zu einer Ebene betrachtet, entlang derer sich der optische Abschnitt (21) erstreckt;
ein Linsenbewegungsmechanismus (13) angeordnet ist, um die Linse (20) quer zur Ebene, entlang derer der optische Abschnitt (21) sich erstreckt, von der Bereitschaftsposition zu einer Einführungsposition zu bewegen, in welcher der Schiebermechanismus (34) die Linse (20) drücken und in das Auge einführen kann; und
das spitze Ende (33a) der Schiebestange (33) außer Eingriff mit der Linse (20) verbleibt und zwischen dem optischen Abschnitt (21) und dem Trägerabschnitt (22) der Linse (20) angeordnet ist, wenn die Linse (20) zu der Einführungsposition durch den Linsenbewegungsmechanismus (13) bewegt wird.

2. Einführungssystem nach Anspruch 1, wobei der Linsenbewegungsmechanismus (13) zwischeneingreifende Vorsprünge (13c) und Vertiefungen (14d, 14e) umfaßt, die Endpositionen des Linsenbewegungsmechanismus (13) definieren, die der Bereitschaftsposition und der Einführungsposition der Linse (20) entsprechen.

3. Einführungssystem nach Anspruch 1 oder 2, wobei das Haltemittel (10) ein Basiselement (11) umfaßt, auf welchem der Linsenbewegungsmechanismus (13) montiert ist und welcher elastisch mit einem Befestigungsabschnitt (35) des Hauptkörpers (31) des Einführungssystems verbunden ist.

## Revendications

1. Dispositif d'insertion pour une lentille intraoculaire (20) ayant une partie optique (21) déformable et au moins une partie (22) de support en forme de boucle, pour supporter la partie (21) optique dans un oeil, le dispositif comprenant :
- des moyens de maintien (10) pour maintenir la lentille intraoculaire (20) en une position d'attente dans laquelle aucun stress n'agit sur la partie optique (21) de la lentille (20) ;
- des moyens de déformation (10,32) pour déformer la lentille (20) en une taille réduite ;
- un tube (32) d'insertion à travers lequel la lentille déformée (20) est insérée dans l'oeil ;
- un mécanisme de poussée (34) ayant une tige de poussée (33) pour pousser la lentille (20) au travers du tube d'insertion (32) le long d'un axe de celui-ci, pour ainsi insérer la lentille (20) dans l'oeil:
**Caractérisé en ce que :**
- Les moyens de maintien (20) sont disposés pour maintenir la lentille (20) dans la position d'attente de façon que la partie (21) d'optique de la lentille (20) soit espacée de l'axe du tube d'insertion (32) et la lentille (20) est espacée de la tige de poussée (33), avec une extrémité pointue (33a) de la toute extrémité (33a) disposée entre la partie optique (21) et la partie de support (22) de la lentille (20) comme montré perpendiculairement à un plan le long duquel la partie optique (21) s'étend ;
- Un mécanisme de déplacement de lentille (13) est disposé pour déplacer la lentille (20) transversalement par rapport au plan le long du quelle la partie tige (21) s'étend à partir de la position d'attente en une position d'insertion en laquelle le mécanisme (34) de poussée peut pousser et insérer la lentille (20) dans l'oeil ;
- La toute extrémité (33a) de la tige de poussée (33) reste hors engagement de la lentille (20) et est disposée entre la partie optique (21) et la partie support (22) de la lentille (20), lorsque la lentille (20) est déplacée vers la position d'insertion par le mécanisme de déplacement de lentille (13).

2. Dispositif d'insertion selon la revendication 1, **caractérisé en ce que** le mécanisme (13) de déplacement de lentille comporte des éléments en saillie (13c) en inter engagement et des dépressions (14d, 14e) qui définissent des positions terminales du mécanisme (13) de déplacement de lentilles correspondant aux positions d'attente et d'insertion de la lentille (20).

3. Dispositif d'insertion selon la revendication 1 ou 2, **caractérisé en ce que** les moyens de maintien (10) comportent un élément de base (11) sur lequel le mécanisme (13) de déplacement de lentilles est monté et qui prend prise élastiquement (11e, 38) avec une partie d'attachement (35) d'un corps principal (31) du dispositif d'insertion.
